# EUROPÄISCHE PATENTSCHRIFT

(11) **EP 1 648 326 B1**
(45) Veröffentlichungstag und Bekanntmachung des Hinweises auf die Patenterteilung: **29.04.2009**
(21) Anmeldenummer: 04762433.3
(22) Anmeldetag: 21.07.2004
(51) Int. Cl.: A61B 19/02, A61L 2/26

(54) **VERWENDUNG EINES FLEXIBLEN ELEMENTS**
USE OF FLEXIBLE LEMENT
UTILISATION D'UN ELEMENT FLEXIBLE

(30) Priorität: 26.07.2003 DE 10334082
(43) Veröffentlichungstag der Anmeldung: 26.04.2006
(73) Patentinhaber: Simmoteit, Robert, 72414 Rangendingen (DE)
(72) Erfinder: Simmoteit, Robert, 72414 Rangendingen (DE)
(86) Internationale Anmeldenummer: PCT/DE2004/001583
(87) Internationale Veröffentlichungsnummer: WO 2005/011513

(56) Entgegenhaltungen:
- WO-A-99/11306
- US-A- 3 285 409
- US-A- 3 464 545
- US-A- 4 262 800
- US-A- 5 004 418
- US-A- 5 346 075
- US-A- 5 346 677
- US-A- 5 918 740

## Beschreibung

Verwendung eines flexiblen elements, insbesondere für die Aufbereitung, Behandlung, Bereitstellung und Transport von Medizinprodukten.

Halterungen aus Kunststoff gehören zum Stand der Technik und werden bei der Produktsterilisierung verwendet. Hierbei handelt es in der Regel um Silikonklemmhalterungen. Diese werden für die Lagerung von empfindlichen medizinische Instrumenten in Siebschalen oder in Trays verwendet. Diese Halterungen müssen der jeweiligen Form der Instrumente speziell angepasst werden, sind sterilisierbar und wiederverwendbar. Derartige Halterungen werden in Siebschalen, Produkttabletts und in Maschineneinsätze verwendet, um medizinische Instrumente sicher zu positionieren. Nachteil dieser Halterungen ist, dass diese bei der Aufbereitung ein Hindernis für Medien und Wellen darstellen und die Produkte im Halterungsbereich schlecht zu reinigen sind. Durch die Temperaturunterschiede sowie durch den verwendeten Mediendruck und physikalischen Verfahren bei der Aufbereitung können die Produkte aus den Halterungen fallen. Technisch ist dieses Problem dadurch gelöst, dass Presshalterungen in Deckel von Trays eingebracht wurden. Diese Ausführungsform hat den gravierenden Nachteil, dass derartige Trays ein schlechtes Reinigungsergebnis besitzen, da die Medien nicht ungehindert die hier eingebrachten Produkte reinigen. Die derzeitigen Halterungen sind rein auf eine Halterungsfunktion ausgelegt und ermöglichen keine aktive Unterstützung der Produktbehandlung im Bereich der Reinigung. Auch die Bereitstellung der Instrumente am Anwendungsort - sprich OP - sind nicht sinnvoll gelöst.

Eine einfache Halterung für Dentalinstrumente ist in der Patentschrift DE 9004079 beschrieben. Dieses Haltersystem ermöglicht zwar eine einfache Produktablage, ist jedoch ungeeignet als Reinigungseinsatz im Bereich der Instrumentenaufbereitung. Eher geeignet sind hingegen Drahtkonstruktionen, da diese sich in Reinigungs- oder Sterilisationsmaschinen einführen lassen. Eines dieser Halterungssysteme in Verbindung mit einem Sterilisationsbehälter ist in der DE 3918147 beschrieben. Jedoch auch hier können die Instrumente aus der Halterung fallen. In der Patentschrift DE 29714090 wird eine 3-Punkt-Lagerung zur sicheren Positionierung von medizinischen Instrumenten beschrieben. Die Instrumente werden in dieser Konstruktion an drei Stellen punktuell auf einer minimalen Fläche gelagert. Nachteilig ist, dass die Halterungen nur eine Instrumentenformen und -größe halten können. Ferner fallen schwere Produkte durch ihr hohes Eigengewicht leicht aus den Halterungen. Nachteilig sind auch die großen stirnseitigen Flächen und großen Befestigungsflächen dieser Halterungen in Verbindung mit einer Trägerstruktur. Diese sich jedoch konstruktionsbedingt und auf Grund der Stabilität notwendig. In der Patentschrift G 94 17 660 wurde versucht die Nachteile der Stabilität durch einen Haltebügel zu verbessern. Durch die verformbare Unterseite können nur kleine Instrumentendurchmesser nebeneinander fixiert werden und der hohe Anpressdruck kann jedoch die eingelagerten Produkte beschädigen oder die aufgelagerten Formteile einen großen flächigen Reinigungsschatten erzeugen. Eine anderen Lösung wurde im US Patent 5 570 794 beschrieben. Über flexible Schlingen eines flachen Bandes werden Gegenstände gelagert. Diese Lagerung ist ungeeignet für unförmige und empfindliche Gegenstände die sich durch die Einlagerung in der Lagerung gegenseitig stark beeinflussen und dadurch beschädigt werden.

Die zweiteilige Form des Anspruchs 1 ist auf US-A-5004418 basiert.

Die beschriebenen Nachteile von Halterungen, die bei der Wiederaufbereitung von Medizinprodukten verwendet werden, sind übertragbar auf andere Produkte und Gegenstände, die einen Aufbereitungs- oder Herstellprozess durchlaufen und sicher transportiert werden müssen. Hierzu gehören beispielsweise Konsumgüter im Hauhaltsbereich, Pharmaprodukte oder gewerbliche Produkte oder Gegenstände die durch den Transport beschädigt werden können.

Die Aufgabe der Erfindung betrifft ein flexibles Element und seine Verwendung, insbesondere für die Aufbereitung, Behandlung, Bereitstellung und Transport von Medizinprodukten. Die Erfindung soll eine flexible Halterung, Lagerung und Sicherung bei einer geringen Halterungsfläche, insbesondere Stirnseitig ermöglichen. Die Erfindung soll durch die Verwendung außerhalb und innerhalb einer Maschine eine verbesserte Reinigung, Desinfizierung, Sterilisierung oder Trocknung oder Handhabung von Produkten und Gegenständen bewirken oder ermöglichen.

Diese Aufgabe wird erfindungsgemäß durch die Verwendung eines flexiblen Elements nach Anspruch 1 gelöst. Die abhängigen Ansprüche betreffen vorteilhafte Ausgestaltungen der Erfindung.

Erfindungswesentlich ist, dass durch Einflechtung und Einpassung oder Einbringung mindestens eines flexiblen Elementes in Verbindung mit einer existierenden Gerüst-, Positions- oder Halterstruktur, Produkte auf einer Struktur, beispielsweise einem Gitter, einem Sieb, einer Platte, einer Wand, einem Tablett, einer Spülleiste oder einem Maschinenteil oder in einem Korb oder Behälter fixierbar sind. Durch das flexible Element kann eine Produkthalterung, eine Produktlagerung oder Produktsicherung oder alles zusammen ermöglichen und dadurch die Produktbehandlung und/oder Produktbereitstellung in oder außerhalb einer Maschine beeinflusst werden. Flexible meint hier in erster Linie, dass das Element auf viele Arten Hand- und Anwendbar, anpassungsfähig, vielseitig einsetzbar und beweglich ist. Wesentlich ist hierbei nicht die Struktur mit der die Gerüst,-, Positions- oder Halterstruktur verbunden ist. Wesentlich ist die Fixierung- und Lagerungsfunktion des flexiblen Elementes in Verbindung mit einer existierenden Gerüst-, Positions- oder Halterstruktur. Von Vorteil kann es sein; wenn die Gesamtkonstruktion tragbar oder beweglich ist. Das flexible Element sichert die Produkte und Gegenstände, beispielsweise medizinische oder technische Instrumente, Geräte oder empfindliche Erzeugnisse, gegen ein Herausfallen oder ermöglicht eine geeignete Lagerungsfläche in Abhängigkeit einer bereits existierenden Gerüstsstruktur - beispielsweise in Form beabstandeter Stifte - oder stellt selbst Halteruhgsflächen oder Halterungspunkte zur Verfügung. Diese Eigenschaften können durch das Material oder die Materialkombination oder über die Materialhärte oder den Aufbau des flexiblen Elementes bestimmt sein. Durch die Verwendung des flexiblen Elementes kann durch Einflechtung und Einpassung oder Einbringung beispielsweise in eine Stiftstruktur, die auch änderbar sein kann, eine geeignete Halterung und/oder Fixierung für Produkte bewirkt werden. Ausformungen oder äußere Oberflächenstrukturen und durch die Form des flexiblen Elementes selbst, kann hierbei die Produktlagerungsfläche, die Anpassungsstruktur oder das Anpressverhaltens des Elementes an die fixierten Produkte und Gegenstände oder in Verbindung mit einer existierende Gerüst-, Positions- oder Halterungsstruktur beeinflussen. Beispielsweise können hier Noppenstrukturen, Oberflächenerhebungen oder Oberflächenstrukturen jeglicher Art die Produktlagerung und -fixierung beeinflussen. Punktuelle Lagerungsflächen oder Produktauflagen können einerseits durch geeignete Querschnittsformen des flexiblen Elementes und anderseits von z. B. kleinsten Oberflächenausformungen, wie z. B. Noppen, kleine Erhebungen, Aufrauhungen oder Fasern auf der Oberfläche des flexiblen Elementes bereitgestellt werden. Alternativ können beispielsweise auch Stifte oder Gerüststrukturen mit Lochstrukturen mit oder ohne Feststellschrauben verwendet werden. Hier kann z. B. ein oder mehrere Silikonbänder feststellbar eingebracht und so unterschiedlichen Produktquerschnitten angepasst werden.

Um kleinste Lagerungsflächen herzustellen, kann z. B. ein flexibles Element in Form einer dünnen Schnurr oder Bandes verwendet werden. Von Vorteil können weiche Auflageflächen sein, wenn empfindliche Produkte aus der Medizintechnik wie z. B. medizinische Augen- oder Dentalinstrumente, in den Halterungen liegen. Die Formbarkeit und Elastizität sowie der Querschnitt des erfinderischen Elementes muss sinnvoll auf die Anwendung abgestimmt und in die existierende auch änderbare Gerüststruktur oder Ähnliches eingeflochten oder eingepasst werden können. Es eignen sich z. B. als Gerüstsstruktur Stifte, die individuell angeordnet werden können, und erst in Verbindung mit dem erfinderischen flexiblen Element eine geeignete Halterung ermöglichen. Insbesondere bei der Fixierung von schweren Gegenständen wie z. B. bei Werkzeugen oder technischen Geräten kann ein biegsames Element, z. B aus Kunststoff oder auch aus Edelstahl oder ein umantelter Draht mit einer hohen Rückstellkraft und/oder Stabilität, von Vorteilt sein.

Die Erfindung ermöglicht wahlweise eine 1-Punkt-, eine 2-Punkt-, eine 1-Flächen oder eine 2 Flächen-Lagerung, aber auch eine Kombination zwischen punktueller und flächiger Lagerung ist in der Halterstruktur möglich. Hierbei sollte die Gerüst- oder Halterungsstruktur in Ihren Ausmaßen größer sein als der Produktquerschnitt sowie im unteren Auflagebereich ein flexibles Element besitzen, damit hier das Produkt entsprechend anliegen kann. Gegenüber einer 3-Punkt-Lagerung erlaubt die Erfindung eine änderbare Lagerung der Produkte in der Halterung mit der Wahlmöglichkeit, die genannten punktuellen und flächigen Auflagen den jeweiligen Bedürfnissen und Prozesserfordernissen anzupassen. Der Anwender kann zwischen dem Produkt und dem obigen flexiblen Element oder auch nach unten hin ein Freiraum lassen, damit während der Reinigung, Desinfizierung und Sterilisierung ein Medium hier durchströmen kann. Im Anschluss an einem dieser Prozesse kann das obige flexible Element auf das Produkt gedrückt werden und eine 2-Punkt- oder 2-Flächen-Lagerung hergestellt werden. Die variable und änderbare Produktlagerung und -sicherung hat noch einen weiteren Vorteil. Die Freiräume und Öffnungen im Halterbereich ändern sich entsprechend der Anordnung der flexiblen Elemente im Halterbereich mit dem Vorteil, dass sich das Produkt während der Prozesse in bestimmten Freiheitsgraden bewegen kann, ohne aus der Halterung zu fallen. Die Beweglichkeit des Produktes in diesem Raum oder Öffnungsbereich ist abhängig vom Aufpressdruck der anliegenden flexiblen Elemente und/oder aus dem Material aus dem das flexible Element besteht. Die genannten Vorteile verbessern z. B. das Reinigungs- und Desinfektions- und/oder Sterilisierungsverhalten und/oder die Entnahme oder die Fixierung der Produkte und Gegenstände aus dem Bereich der Medizintechnik im Halterbereich.

Die Erfindung erlaubt weiter eine physikalisch beispielsweise eine manuelle beeinflussbare einfache Fixierung von Produkten durch Zug und Druck über das flexible Element. Die Anpresskraft des flexiblen Elementes an die Gerüststruktur des Halters ist im bestimmten Rahmen und Grenzen änder- bzw. beeinflussbar. Änderbar in der Art, dass auf das flexible Element mit elastischen Eigenschaften ein Zug z. B. in Längsrichtung angelegt werden kann. Die Größe der Anpresskraft wird beeinflusst durch die Elastizitäts- und Verformungseigenschaften des flexiblen Elementes. Vorzugsweise durch ein elastisches, dehnbares oder biegsames Element kann eine Kraft oder ein Druck von oben auf das zu fixierende Produkt ausgeübt werden. Diese Kraft oder dieser Druck von oben ist abhängig von der Anpresskraft an die Gerüststruktur und von der Verformungsstabilität des flexiblen Elementes. Die Festigkeit dieser physikalischen Verbindung ist darüber hinaus vom Formschluss der anliegenden Flächen, von der Oberflächenrauhigkeit und den Oberflächenstrukturen sowie von der Art der Ausformungen der Gerüststruktur und von den Elementeigenschaften mit beeinflusst. Hierbei können Oberflächenstrukturierung und/oder Oberflächenbeschichtungen bis in den Nanobereich zum Einsatz kommen. Mögliche Alternativen sind Strukturierungen ähnlich wie bei einem Klettverschluss oder faserartige Aufrauungen oder Strukturen, wie sie in der Natur vorkommen, um eine physikalisch stabile Verbindung mit einer Gerüststruktur herzustellen. Das heißt, der mögliche von oben erzeugte Druck ist kleiner oder gleich der Kraft, mit der das Element in der Gerüststruktur des Halters bewegt werden kann.

Die mechanische Stabilität der Verbindung zwischen dem flexiblen Element und der Gerüststruktur wird durch die Größe der Reibungskraft, der Materialeigenschaft und der Art der Verformung des flexiblen Elementes bestimmt. Dies gilt auch für den Fall, wenn die Verbindung sich in eine bestimmte Richtung wieder lösen soll. Die Reibungskraft ist unabhängig von der Größe der Berührungsfläche und wirkt stets parallel zur Berührungsfläche und ist der Bewegung entgegengerichtet. Befindet sich z. B. ein medizinisches Instrument in der Halterung, so kann von oben der Aufpressdruck des flexiblen Elementes, vorzugsweise ein in sich stabiles oder durch Zug stabilisiertes Element, manuell in bestimmten Grenzen oder stufenweise durch Aufdrücken und/oder unterstützt durch geeignete Führungsteile ein- oder nachgestellt werden. Je nach Art, Empfindlichkeit und Form des Produktes oder des Instrumentes wird individuell eine sichere Einklemmung bzw. Lagerung hergestellt und z. B. das Instrument sicher vor ein Herausfallen in der Halterung verankert. Die Sicherung sollte so gut sein, dass das Instrument nicht aus der Halterung fällt, wenn dieses z. B. über Kopf oder schräg gehalten wird. Die Beeinflussung der Produktfixierung ist von Bedeutung bei der Aufbereitung von Gegenständen jeglicher Art. Beispielsweise werden Produkte in einer Reinigungsmaschine starken Medienstrahlen ausgesetzt und können sich so aus den Halterungen lösen. Die Erfindung kann beispielsweise in einer Maschine oder auf Maschinenteilen z. B. in Verbindung mit einem Spülmaschineneinschubwagen einer Spülmaschine oder hier vorhandenen Spülleisten oder in Instrumentensiebkörben angewendet werden. Das flexible Element ermöglicht in Verbindung mit der Gerüststruktur oder mit Teilen des Wagens eine stabile Fixierung von Produkten. Insbesondere von unförmigen Produkten wie z. B. von Instrumenten, Besteck und/oder Geschirr wie Teller, Tassen und Gläser sowie anderen Produkten. Alternativ kann Aufbereitungszubehör außerhalb der Spülmaschine zum Transport z. B. in Form von Siebkörben, Gestellen oder Sterilcontainern oder als Tablett auch in Sterilisationsmaschinen einsetzbar aber auch fester Bestandteil einer Maschine sein.

Das flexible Element ist austauschbar und besitzt z. B. elastische, formbare oder biegsame Eigenschaften. Es kann einfach in vorgegebene Ausformungen der Gerüst-, der Positions- oder der Halterstruktur eingepasst oder eingebracht werden. Von Vorteil kann es hierbei sein, dass eine speziell großzügig vorgeformte Halterungsstruktur ausgebildet ist, die beispielsweise im oberen Halterungsbereich Aussparrungen, Ösen, Klemmstrukturen, Einkerbungen, Kugelaufsätze, Führungsteile oder andere Ausformungen besitzen kann. Diese Ausformungen und Anordnungen der Gerüststruktur sollen das Einlegen und Herausnehmen des flexiblen Elementes gewährleisten und insgesamt die Funktion des flexiblen Elementes unterstützen. Weiter können die Ausformungen so gewählt sein, dass insbesondere das Herauslösen des flexiblen Elementes ohne besondere mechanische Hilfsmittel oder ohne starken Zug möglich ist. Gleiches gilt für das Einlegen des flexiblen Elementes in die Gerüststruktur des Halters. Insbesondere Stift- oder Drahtstrukturen eignen sich als Gerüstsstrukturen. Stifte dadurch, dass diese unterschiedlich voneinander und individuell beabstandet sein können und nachträglich änderbar sind.

Die Erfindung ermöglicht eine individuell auf das jeweilige Produkt abgestimmte änderbare Produktsicherung oder Produktlagerung, wobei sich das flexible Element optimal in bestimmten Grenzen an die Produktform anpasst. Weiter ermöglicht die Erfindung die Herstellung und Einhaltung hygienischer und anwenderbezogener Anforderungen auf höchstem Niveau, so wie sie bei der Produktaufbereitung von Instrumenten in der Medizin gefordert werden. Hierzu gehört z. B. auch die Schonung und Sicherung der Produkte während des Transportes und die Einhaltung der Sterilitätskriterien bei der Entnahme. Falls das flexible Element stark verschmutzt ist, kann dieses verworfen werden. Dieses ist möglich, weil das Element ein leicht auswechselbarer, preiswerter Bestandteil der Halterung ist. Die Flexibilität der erfinderischen Elemente ermöglicht in Verbindung mit Positionsstrukturen eine höhenverstellbare Halterung bzw. Lagerungsfläche - ohne die Erfindung zu verlassen - in der Art, dass mit einem unteren Element als Lagerungsfläche und mit einem oberen beabstandeten Element als Produktsicherung eine Halterung hergestellt wird. Über beispielsweise eine Stiftstruktur, kann das obere aber auch das untere Band oder Element stufenweise durch die vorhandenen Einkerbungen oder stufenfrei verstellt werden. Das Produkt in dieser Halterung befindet sich in einer Art Schwebezustand und berührt dabei nicht den Boden oder die Oberfläche z. B einer Trägerstruktur. Medien können über Öffnungen in der Halterung oder unter und über das flexible Element strömen. Die hier so hergestellten Halterungen sind über den definierten Bereich der Positionsstrukturen z. B. in Form von Metallstiften insgesamt höhenverstellbar. Das obige Element kann leicht herausgelöst werden und gibt das Produkt in der Halterung zur Verwendung frei. Diese Ausführung ist insbesondere dort wichtig, wo eine sehr geringe stirnseitige Fläche von Vorteil ist und die fixierten Produkte gut sichtbar sein oder zugänglich sein müssen.

Das flexible Element kann als Band, Draht, Hohlwelle oder Röhre z. B. als Schlauchelement ausgeformt sein. Das Element kann z. B. in seinem Kern eine Stahlspirale, einen Metall- oder einen Memory-Werkstoff, eine Glasfaser oder elektronische Teile wie Sensoren enthalten. An den Enden können Formelemente zur besseren Handhabung oder z. B. flache Transponder-Label angebracht sein, um den Behandlungsprozess oder eine Produktidentifizierung über den Raum hinweg zu ermöglichen. Dabei kann es von Vorteil sein, wenn das endständige Formteil auf dem flexiblen Element verschiebbar ist, und über eine Fixierung z. B. eine Schraube feststellbar ist. Dieses Formelement kann so ohne Kürzung des flexiblen Elementes eine lösbare und adaptierbare Verbindung mit einer Halterung eingehen. Das verwendete Material sollte entsprechend des Einsatzbereiches im Bereich der medizinischen Aufbereitung innert gegenüber chemischen Lösungen wie z. B. Reinigungslösungen temperaturstabil und wiederverwendbar sein. In Ausbildung einer Röhre, wie beispielsweise in Form eines Schlauches aus flexiblen, elastischen oder biegsamen Material, ermöglicht das Element die Durch- oder Ableitung von Medien. Ein Glasfaserkern hingegen ermöglicht das Einbringen von Licht, ein Stromkabel das Einbringen von Strom. Sensoren in Verbindung mit einem Stromkabel im Kern können genutzt werden, um Temperatur oder andere physikalische Parameter in unmittelbarer Nähe der Halterungen zu ermitteln. Von Vorteil ist, wenn das erfinderische Element aus medizinischen Werkstoffen oder Materialien besteht. Hierbei kann das Element zum Teil oder ganz aus Metall, aus Glas oder aus Silikon, PTFE, Gummi, Polyurethan oder anderen Kunststoffen bestehen. Alternativ kann es aus einem Memory-Material mit einem Formgedächnis oder aus einem Material mit unterschiedlichen Zustandsformen bestehen. Um dem Element stabilisierende Eigenschaften zu geben, können im Element metallische Werkstoffe in Form von Spiralen, Hohlwellen oder Federn verarbeitet sein. Es sollte jedoch bei der Anwendung in der Medizin sichergestellt sein, dass durch den Abrieb von Material durch medizinische Instrumente kein Risiko oder gar eine Gesundheitsschädigung für den Patienten entsteht.

Die Erfindung unterstützt beispielsweise als röhrenförmiges Element nicht nur die effektive Reinigung oder Behandlung von Produktflächen durch Reduzierung der Reinigungs- oder Behandlungsschatten bei minimierter Produktauflage und Frontfläche, sondern kann auch weitergehende Aufgaben übernehmen. Durch Anbringen von Adaptern, wie z. B. Lour Look Anschlüssen, können hieran angeschlossene Hohlraumprodukte direkt von Medien durchströmt werden. Diese Durchspülung kann außerhalb oder in eine Maschine erfolgen. Über das flexible Element können Funktionen wie beispielsweise Medien oder Strom oder Lichtwellen eingebracht werden und so die Behandlungen der Produkte unterstützen oder andere Prozesse ermöglichen. Von Vorteil ist, dass Medien, Wellen oder andere physikalische Parameter in unmittelbarer Produktnähe eingebracht werden können. Wesentlich ist hierbei, dass sich durch Anordnung der flexiblen Elemente die Produkte so positionieren lassen, dass z. B. die Ultraschaltwellen oder Behandlungsmedien auch schlecht zugängliche Produktoberflächen erreichen. Die Verwendung des flexiblen Elemente stellt in Kombination mit z. B. einer dünnen Drahtstruktur kein großes Hindernis für die Medien dar und behindern nur geringfügig die Behandlung mit Wellen. Mit derartigen Strukturen lassen sich auch kleinste Auflageflächen im Halterungsbereich realisieren.

Eine bevorzugte einfache Ausgestaltung des flexiblen Elementes besteht aus einem Silikon-Band oder einem Silkon ummantelten Draht. Je nach Härtegrad und Art des Silikons ändern sich auch dessen Eigenschaften, wie z. B. Biegsamkeit, Elastizität und Anpassungsfähigkeit. Eine runde Querschnittsform des Elementes, kann bei der Herstellung von Vorteil sein. Alternativ kann der Querschnitt quadratisch oder andere geeignete Querschnittsformen besitzen. Im Falle eines Drahtes kann das Element entsprechend der Gerüststruktur vorgeformt sein oder geeignete Oberflächenstrukturen aufweisen. Ein vorgeformtes Edelstahlelement lässt sich leicht von oben einpassen und durch die Art der Halterung, z. B. durch Verjüngung der Struktur nach unten, einpressen. Die benötigte Länge sollte auf die Gerüststruktur der Halterung abgestimmt sein. Von Vorteil kann es sein, wenn die Länge durch Abschneiden nachträglich reduzierbar ist. Alternativ kann das Material transparent oder farbig sein und z. B flureszierende Farben enthalten, um ein Farbleitsystem zu ermöglichen. Flureszierende Eigenschaften verbessern die Handhabung in abgedunkelten Räumen. Diese Ausgestaltung kann für die Produktlagerung oder für die Produktsicherung oder in einer Kombination benutzt werden. Die Durchmesser der flexiblen Elemente können sich gemäß der Funktion technisch oder produktbezogen im oberen oder unteren Anwendungsbereich unterscheiden.

In der weiteren vorteilhaften Ausgestaltung besitzt das Element mindestens eine endständige Struktur, um eine Verankerung oder das Einklemmen zu ermöglichen. Diese können Kugeln, verschiebbare Hülsen mit Fixierschrauben, flächige Ansatzstücke oder eine andere Ausformung besitzen. Von Vorteil kann es sein, wenn diese Endstücke individuell angebracht werden können, beispielsweise durch Verklebung, Verschweißung, mittels einer Presspassung oder durch eine andere Verbindungsart. Alternativ können die endständigen Strukturen beweglich oder feste Bestandteile der Elemente sein oder auch austauschbare oder fest integrierte elektrische Komponenten wie z. B. Transponder oder Sensoren enthalten.

In einer weiteren bevorzugten Ausgestaltung besteht das Element aus einer flexiblen Röhre oder einem Schlauch oder einer stabilen Röhre mit flexiblen Teilen. Von Vorteil kann es hierbei sein, dass die Röhre aufgrund der dünnen Wandstruktur aus Edelstahl besteht. Diese flexible Röhre sollte vorgeformt sein, um entsprechend leicht eingepasst zu werden. Die Röhren sollen für den Mediendurchtritt offen sein. Über eine Schnellverbindung kann Medium in das Element direkt an die gelagerten Produkte herangeführt werden. Diese können über Öffnungen im Schlauch oder der Röhre nach außen treten und beispielsweise die Reinigung unterstützen. Alternativ kann Medium über Anschlusstücke oder Adapter, die am flexiblen Element angebracht sind, das Innenlummen von Instrumenten reinigen.

Es kann bevorzugt sein, dass anstelle einer Röhre, Schlauch oder Bandstruktur ein flächiges Element mit zusätzlichen Formelementen verwendet wird. Die Formelemente können so ausgeformt sein, dass diese in die Halterung eingedrückt oder eingepresst werden kann. Damit werden zwei Halterungs- oder Fixierungsarten in einem Element vereinigt. Eine Halterungs- oder Fixierungsart besteht im adaptierten Formschluss zwischen der Gerüststruktur des Halters durch die Formelemente. Eine sinnvolle Halterungs- oder Fixierungsart erfolgt durch Einflechtung oder Einbringen des flexiblen Elements in beabstandete Stifte als Gerüststruktur in Verbindung mit einer Trägerstruktur die auch ein Spülrohr sein kann und damit verbunden die individuelle und änderbare Positionierung des Elementes ermöglicht.

Bei einer weitergehenden Ausgestaltung der Erfindung besteht das flexible Element aus einem Lichtleiter mit dem Licht eingestrahlt wird. Bei UV-Licht, wird im Halterungsbereich und über dem Raum hinweg eine Sterilisierung ermöglicht. Alternativ können im Element oder endständig elektronische Teile eingebracht sein, um hiermit Temperatur- oder Druckdaten ermitteln zu können. Andere elektronische Komponenten wie die von Transpondern ermöglichen die Datenkommunikation über den Raum hinweg.

In einer weiteren Ausgestaltung wird ein Memory-Werkstoff verwendet. Das Memory-Material des Elementes besitzt z. B. unterschiedliche Temperaturzustände und/oder ein geeignetes Formgedächnis. Im Gebrauchszustand (15 - 30 °C) ist es besonders biegsam und elastisch.

Hingegen bei höheren Temperaturen formbeständig. Letzteres verhindert bei hoher mechanischer Beanspruchung ein Herauslösen des Elementes. Alternativ unterstützt der Memory-Werkstoff das Einbringen des Elements in die Gerüststruktur der Halterung in der Art, dass das Material bei der Erstanwendung sich die Form der Gerüststruktur des Halters gemerkt hat. Die eingenommene Form sollte hierbei die anderen Eigenschaften des Elementes nicht maßgeblich beeinflussen oder die Handhabung behindern.

Eine gebräuchliche Ausgestaltung der Erfindung kann die Anwendung in Siebschalen, Metallgewebe oder Metallgitter sein. Über einfache Abstandhalter bzw. Positionsstifte, z. B. mit einem Kugelkopf oder einer Drahthalterung aus Edelstahl oder Kunststoff mit geeigneter Ausformung, können Instrumente auf dem Sieb bzw. Instrumentenkorb über mindestens ein flexibles Element fixiert werden. Die Anordnung dieser Stifte oder Halterungsgerüststruktur kann geometrisch, produktbezogen oder individuell verteilt sein. Mittels eines Elementes oder mehrerer Elemente werden die hier eingebrachten Instrumente anwendungsbezogen fixiert, d. h. auf die spätere Anwendung hin beispielsweise im OP oder in der Arztpraxis. Vorzugsweise werden die Instrumente in zwei parallele Bänder geschoben oder hier wieder herausgeschoben. Diese Instrumente können einerseits einfaches chirurgisches Besteck wie Scheren, Pinzetten oder Nadelhalter, aber auch komplizierte Endoskope oder MIC-Instrumente bis hin zu empfindlichen Augeninstrumenten sein. Individuell können zusätzliche flexible Elemente eine weiche Auflage im Sieb ermöglichen, um eine Beschädigung beim Transport oder die Entnahme der Produkte aus dem Sieb zu verbessern. Beispielsweise können hier die Produkte so angeordnet werden, dass die Entnahme entsprechend dem Verwendungszweck angepasst ist. Alternativ kann als Sieb oder Gitter eine Kunststoff-, Metall- oder eine Siebschale oder -korb bzw. als Platte ein Tablett die flächige Auflage bilden. Wichtig ist hierbei, dass geeignete Gerüststrukturen oder Positionsstifte vorhanden sind, die eine Einflechtung der Elemente ermöglichen. Insgesamt von Vorteil ist, wenn zwei parallel verlaufene Elemente oder Bänder sich nach oben und gleichzeitig nach unten bewegen können und so die Flexibilität und Anpassung der ausgebildeten Halterungsstruktur erhöhen.

Die Erfindung unterstützt und ermöglicht Anwendungen zur Produktbehandlung und/oder Produktbreitstellung durch geeignete Lagerung. Beispielsweise die Behandlung mit Medien und mit physikalischen Wellen, wie z. B. mit Licht oder Ultraschallwellen oder alternativ die Entnahme unter sterilen Bedingungen. Gut gesicherte medizinische Instrumente erlauben eine direkte Positionierung hin zur Behandlungsquelle. Von Vorteil ist z. B. eine über Kopflagerung oder eine aufrechte Positionierung. Die Produkte dürfen nicht durch ihr Eigengewicht oder durch den Medien- oder Schalldruck aus der Halterung fallen. Die Medien oder Wellen erreichen alle Produktoberflächen, auch die Hinteren Produktflächen in den Halterungen besser, wenn sich diese bewegen. Um Produkthohlräume behandeln zu können benötigen diese Zusatzeinrichtungen zum Durchströmen mit Medium. Die Lagerung in unterschiedlichen Positionen bei einer herstellbaren geringen Halterungsfläche, insbesondere stirnseitig, begünstigt Aufbereitungs- oder Bearbeitungsprozesse und reduziert Behandlungsschatten. Hierzu gehören Aufbereitungsprozesse wie z. B. die Reinigung, die Desinfizierung, die Sterilisierung sowie der Transport. Die Erfindung ermöglicht ferner eine manuelle und maschinell unterstützte Medienzufuhr über flexible Elemente mit Hohlräumen über Öffnungen, Adapter, Konnektoren oder Ventile in und um das zu behandelnde Produkt in der Halterung. Bevorzugt ist hierbei, wenn sich während der Behandlung z. B. in einer Reinigungsmaschine oder allgemein bei der Produktaufbereitung in der Medizin das Produkt in der Halterung durch den Mediendruck oder Schalldruck bewegt. Durch die Beweglichkeit der Produkte innerhalb der Halterung wird weiter die Verletzungsgefahr von bereits benutzten Produkten bei Operationen und damit die Infektionsgefahr reduziert. Die fixierten medizinischen Instrumente z. B. zwischen zwei flexiblen Silikonbändem geben nach und vermindern so die Gefahr von Stichverletzungen.

Die Reinigung, Desinfizierung und Sterilisierung kann auch dadurch unterstützt sein, dass Medium oder Licht bei der manuellen Vorbehandlung sowie zusätzlich zur maschinellen Aufbereitung im Halterungsbereich eingebracht wird. Durch direkte Zuführung von z. B. Wassstoffperoxid über ein flexibles Element in das zu sterilisierende Produkt wird die Sterilisierungssicherheit erhöht. Bei der Verwendung von Licht können chemische Reaktionen in Gang gesetzt werden, welche die Reinigungs- oder Desinfizierungsleistung der verwendeten Medien verbessern. In Ausprägung eines Lichtleiters kann UV-Licht über das erfinderische Element zur Abtötung von Keimen, Bakterien oder Zellen besonders effektiv im Halterungsbereich eingestrahlt werden. Weiter erlaubt ein röhrenförmiges Element das Einbringen von Druckluft über Austassöffnungen. Dieses kann so produktnah über die Instrumentenoberfläche zur Trocknung geführt werden, aber auch Innenlumen der Produkte von Lösungsmittelresten befreien. Die Erfindung unterstütz beispielsweise verfahrentechnische Anwendungen durch elektronische Komponenten, die im flexiblen Element oder endständig vorhanden sein können. Insbesondere ermöglichen Transponder eine Produktidentifizierung oder eine Behandlungsüberwachung. Eine Überwachung wird durch den Einsatz von Sensoren ermöglicht. Diese steuern z. B. über dem Temperaturverlauf, Prozesse während der Behandlung in einer Maschine. Durch die Anwendung des flexibeln Elementes in Kombination mit Aufbereitungszubehör wie Einschubwagen werden Produkte sicher positioniert und die Validierung dieser Produkte unterstützt. Aufbereitete Medizinprodukte können so ohne umpacken auf einem Träger verbleiben und nach einer Sterilisierung in einem Sterilcontainer in den OP transportiert werden. Dadurch, dass das z. B. das obere flexible Element einfach und schnell durch leichten Zug Entfernbar sein kann, können die Produkte steril entnommen werden. Alternativ werden die Instrumente nur herausgeschoben und können auf das obere Band nach Gebrauch wieder abgelegt werden. Im Umkehrschritt erfolgt die Einlagerung durch einschieben der Instrumente z. B. zwischen zwei Bändern. Die Bereitstellung und Handhabung steriler Produkte, beispielweise im OP, ist hierbei erleichtert und unkompliziert.

Die Erfindung kann auf alle Lebensbereiche sowie auf gewerbliche Anwendungen übertragen werden, wo Produkte in Halterungen gesichert und gelagert werden müssen. Insbesondere überall dort, wo Produkte transportiert oder gesichert oder fixiert werden müssen und es darauf ankommt die Produktentnahme aus der Halterung auf einfachste Art zu ermöglichen. Überall auch dort, wo es gilt, eine wirtschaftliche Bereitstellung und Behandlung der Produkte in sogenannten Produkt-Sets zu gewährleisten.

Weitere Vorteile der Erfindung ergeben sich aus der Wahl des Einsatzgebietes, der eingesetzten Behandlungsmethoden sowie durch eine wirtschaftliche Verwendung der eingesetzten Materialien und aus der Beschreibung und der beigefügten Zeichnung.

Es versteht sich, dass die vorstehenden genannten und die nachstehenden noch zu erläuternden Merkmale nicht nur in der jeweils angegebenen Kombination, sondern auch in anderen Kombinationen oder in Alleinstellungen verwendbar sind, ohne den Rahmen der vorliegenden Erfindung zu verlassen.

Ausführungsbeispiele der Erfindung sind in der Zeichnung darstellt und werden in der nachfolgenden Beschreibung näher erläutert. Es zeigen:

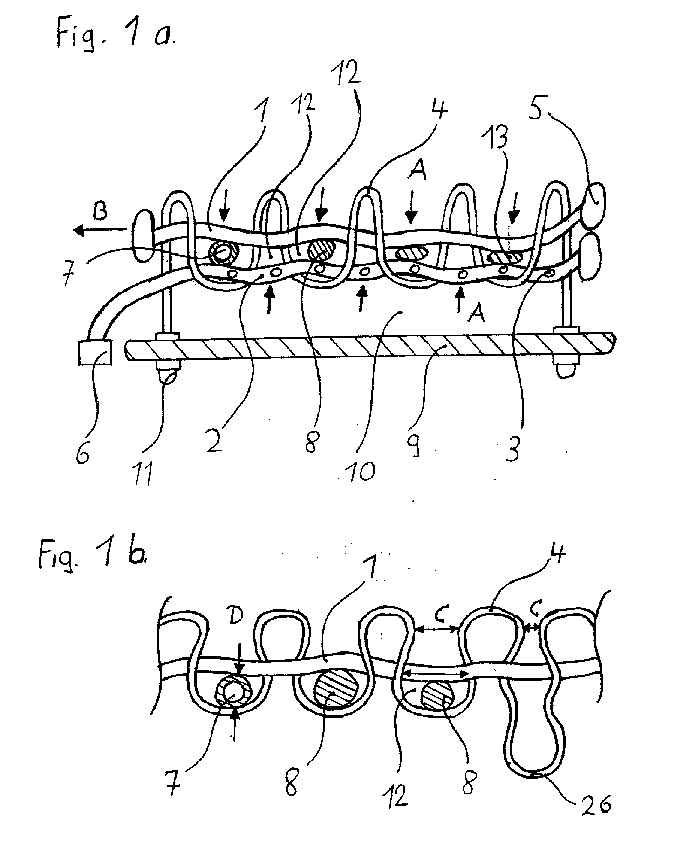

- Fig. 1a: Detaillierte Schnittdarstellung der Erfindung in Kombination mit einer Drahthalterung als Gerüststruktur und einer Platte.
- Fig. 1 b: Detaillierte Schnittdarstellung in Kombination mit einer ausgeformten Drahthalterung.
- Fig. 2a.: Draufsichten möglicher Einflechtungen der flexiblen Elemente.
- Fig. 2b.: Detaillierte Ansichten der Erfindung mit zwei beabstandeten eingeflochtenen flexiblen Elementen.
- Fig. 2c.: Detailansichten von mindestens einem eingebrachten Element in Verbindung mit einer Positionsstruktur.
- Fig. 3a.: Schnittdarstellung eines flexiblen Elementes mit einem Kern
- Fig. 3b.: Detaillierte Schnittdarstellung einer Drahthalterung als Gerüststruktur mit einem Produkt in einer 1-Punkt-1 Flächen-Lagerungsposition
- Fig. 4.: Schematisches flexibles Element mit zusätzlichen funktionalen Formelementen
- Fig. 5a.: Detaillierte Schnittdarstellung einer Gerüststruktur eines Halters mit einem flexiblen Element zum Durchspülen von Instrumenten
- Fig. 5b.: Detaillierte Schnittdarstellung einer Spülleiste zum durchspülen von Instrumenten mit einem flexiblen Element als Instrumentensicherung
- Fig. 6: Draufsicht einer Siebplatte mit fixierten geometrisch angeordneten Positionsstiften bestückt mit chirurgischen Instrumenten
- Fig. 7: Schnittdarstellung einer Halterung bei der das Element eine flexible Produktlagerung und -sicherung in Kombination mit Positionsstiften ermöglicht.
- Fig. 8: Reinigungseffizienz von Augeninstrumenten die mit der Bandhalterung gelagert wurden.

Figur 1a. zeigt eine detaillierte Schnittdarstellung der Erfindung in Kombination mit einer Drahthalterung als Gerüststruktur und einer Platte. Hierbei umwinden zwei flexible Elemente **1** und **2** eine Drahtgerüststruktur **4,** welche die Struktur des Halters vorgibt. Die hier dargestellte Einflechtung bzw. physikalische Verbindung der Gerüststrukturen **4** durch die erfinderischen Elemente **1** und **2** stellt nur eine mögliche Anordnung dar und kann individuell vom Anwender gewählt und manuell eingebracht werden. An den Enden beider Elemente **1** und **2** befindet sich ein Formteil **5.** Das Formteil **5** soll verhindern, dass die Elemente **1** und **2** sich aus der Drahtgerüststruktur **4** lösen. Alternativ könnte das Element durch einen Sicherungsverschluss in der Gerüststruktur zusätzlich festgestellt werden. Ferner kann das Formteil **5** so ausgeformt sein, dass man hiermit Zug auf das flexible Element **1** und **2 in** Längsrichtung ausüben kann. Eine mögliche Zugrichtung ist mit dem **Pfeil B** angezeigt. In dieser erfinderischen Anordnung ist als Produktlagerung bzw. -auflage ein flexibles Element **2** z. B. in Form eines Silikonschlauches, aber auch in Form einer Edelstahlröhre möglich. Auf der Produktlagerung **2** können insbesondere empfindliche Produkte wie z. B. Augeninstrumente weich gelagert werden. In der Schnittdarstellung sind Produktquerschnitte mit einem Hohlraum **7** oder mit runden **8** oder ovalen **13** Querschnitt dargestellt. Zum Durchleiten von Medien befindet sich am Schlauchende des unteren Elements **2** ein Adapter oder eine Schnellkupplung **6,** z. B. für den Anschluss an einem Wasserhahn oder einen Pumpenschlauch. Über das Element **2** können Medien über Austrittsöffnungen **3** direkt in die Nähe der hier gelagerten Produkte transportiert werden. Durch Einbringen beispielsweise von Sensoren kann in unmittelbarer Nähe die Temperatur ermittelt werden. Die auf dem flexiblen Element **2** aufliegenden Produkte **7, 8** und **13** können durch ein flexibles Element **1** fixiert werden. Durch manuelles Andrücken - simuliert durch die **Pfeile A -** werden die Produkte zwischen den Elementen **1** und **2** eingeklemmt. Es entsteht eine 2-Flächen-Lagerung für das Produkt mit einer änderbaren Öffnung **12.** Alternativ kann über das untere flexible Element **2** die Höhe - simuliert durch die **Pfeile A -** der Halterung verstellt und damit das Produkt weiter von der Oberfläche einer Unterkonstruktion oder Trägerkonstruktion z. B. einer Platte **9** entfernt werden. Hier kann sich das Produkt während der Behandlung bewegen. Diese Bewegung ist Vorteilhaft für die Produktaufbereitung. Die Flexibilität des Elementes erlaubt hierbei das Einklemmen unterschiedlicher Querschnittsformen und Querschnittsgrößen. Der Aufpressdruck ist kleiner oder gleich der Kraft, mit der das Element in der Gerüststruktur des Halters bewegt werden kann. Der hier dargestellte Instrumentenhalter aus einem Drahtgerüst **4** ist über eine Schraubverbindung **11** mit einer Platte **9,** z. B. in Form einer Lochplatte verbunden. Alternativ kann hier auch ein Sieb, Gitter oder ein Metallgeflecht zu Einsatz kommen. Diese Konstruktion kann beispielsweise bei der Instrumentenaufbreitung im Bereich der Reinigung, Desinfizierung und Sterilisierung vorteilhaft eingesetzt werden, insbesondere durch die kleine Stirnfläche der gesamten Halterung. **12** und **10** stellen Durchlässe für Medien oder Wellen in Medien dar. Die hergestellte Öffnungen **12** in der Halterung zwischen den elastischen Elementen **1** und **2** sind ebenso gut durchlässig wie die Öffnungen **10** unterhalb des Halters.

Figur 1b. zeigt eine detaillierte Schnittdarstellung in Kombination mit einer ausgeformten Drahthalterung. In dieser Ausformung ist ein flexibles Element **1** in einem Drahtgerüst **4** eingeflochten, bei dem sich die Halterung nach oben hin verjüngt. Diese Verjüngung ist durch **Pfeile C** angezeigt. Diese Ausformungen der Gerüststruktur des Halters erhöhen den Formschluss zwischen dem flexiblen Element **1** und der Gerüststruktur **4.** Das Element **1** sitzt so stabiler in dem Drahtgerüst **4** und hält durch die Verjüngung nach oben die Produkte **7** und **8** bester fest. Ferner ist dargestellt, dass mit dem Element **1** unterschiedlich große Produktquerschnitte von Produkten **8** fixierbar sind. Die Halterungsstruktur **4** muss nur entsprechend groß ausgelegt sein. Die Öffnung **12** ist so groß, dass die in der Halterung eingeklemmte Produkte **7** und **8** sich trotz einer 2-Punkt-Lagerung bewegt können und der Durchtritt von Medien oder Wellen möglich ist. Die Ausbildung der 2-Flächen-Lagerung ist durch die **Pfeile D** gekennzeichnet. Zur Stabilisierung der Halterung z. B. nach unten, besitzt die Drahtgerüststruktur **4** eine verlängerte Ausformung **26** mit einer obigen Verjüngung, hier mit einem **Pfeil C** gekennzeichnet. Von oben kann das elastische Element **1** nachjustiert werden und ist so zusätzlich gegen ein Herausfallen gesichert.

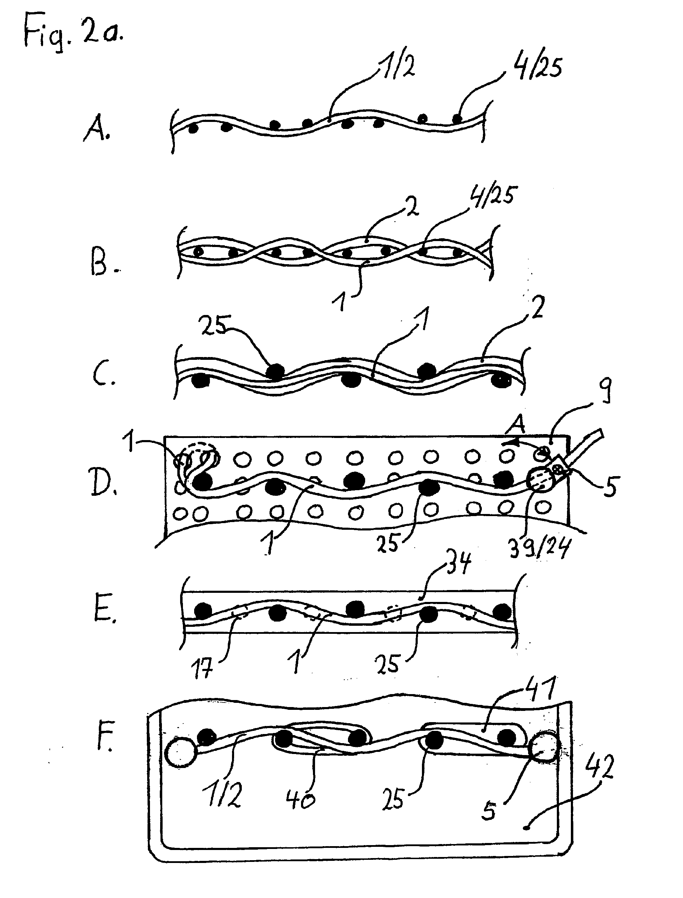

Figur 2a. zeigt Draufsichten möglicher Einflechtungen der flexiblen Elemente. In den Abbildungen A, B, C, D, E und F sind mögliche Verformungen der Elemente **1** und **2** und Verflechtungen mit einer Gerüststruktur **4** oder **25** auf oder ohne eine Struktur **9** dargestellt. Mit **4** oder **25** sind hier z. B. Positionsstifte **25** und mögliche Anordnungen von Stifte **25** oder Drahtstruturen **4** dargestellt. In der Abbildung A umwindet ein oberer **1** und/oder unteres flexibles Element **2** alternierend jeweils zwei Stifte **25.** Alternativ kann unterhalb des flexiblen Elementes **1** ein zweites gleichgroßes flexibles Element **2** in gleicher Anordnung vorhanden sein. In Abbildung B umwinden zwei gleichgroße flexible Elemente **1** und **2** in entgegengesetzter Richtung alternierend und wellenförmig wie ein Frequenzband die Drahtstruktur **4** oder die Stifte **25.** Das flexible Element **2** in der Abbildung C besitzt einen größeren Querschnitt als das Element **1.** Beide Elemente **1** und **2** umwinden die Stifte **25** in gleicher Art und Weise. Die Größe des Elementes **2** kann z. B. dadurch begründet sein, dass dieses Element ein Schlauch oder Röhre mit einem großem Lumen besitzt, durch das die benötigte Medienmenge strömen kann. Abbildung D zeigt eine gleiche Anordnung der Stifte **25** wie in C, jedoch in Verbindung mit einer Struktur **9** in Form einer Lochplatte. Hierbei windet sich ein langes flexibles Element **1** oben und unten, z. B. in Ausprägung eines Silikonbandes hin (unten) und zurück (oben) um die Stifte **25** und wird an einem Ende in die Lochstruktur der Lochplatte **9** eingefädelt. Endständig besitzt das Band ein Verschlussteil oder -ring **5,** welches in eine Halterung **39** oder **24** eingebracht wird. Das endständige Formteil **5** besitzt hier die Form eine beweglichen Hülse, die verschiebbar ist, mit einer Feststellschraube. Dadurch kann, ohne das flexible Element **1** zu kürzen, die Stabilität der Verbindung mit der Halterung **39** und **24** ein- oder nachgestellt werden. **Pfeil A** soll verdeutlichen, dass das so eingebrachte Band 1 in die Halterung **39** ein und aus geführt werden kann. Zwischen dem unteren Teil, welches die Ablagefläche bildet, und dem oberen Teil des Bandes **1,** welches die Produktsicherung bildet, kann beispielsweise ein Produkt in einer 1-Punkt-1Flächen-Lagerungsposition eingeklemmt werden. Weiterführend zeigt die Abbildung E ein Spülrohr **34** mit Stiften **25** in dem ein Element **1** oder Band eingefädelt ist. Das oben geführte Band **1,** fixiert z. B. Hohlrauminstrumente die hier an die vorhandenen Adapter **17** angeschlossen sind. So können die Instrumente nicht nach oben durch den Spüldruck herausgedrückt werden. Die letzte Abbildung F. zeigt wiederum eine Stiftstruktur von beabstandeten Stiften **25** in Kombination mit elastischen Ringen **40** oder anderen Formelementen **41.** Diese Ringe **40** oder Formelemente **41** können die untere Produktauflage herstellen oder zur besseren Einklemmung der hier eingebrachten Instrumente dienen. Die Stifte **25** sind hier auf dem Boden eines Tabletts **42** oder alternativ auf einer Instrumentensiebschalle oder auf einen anderen Platte mit einer Randstruktur angeordnet. Durch die Anpresskraft des Elements **1** bzw. **2** an die Stifte **25** könne diese sicher gehalten werden. Diese Anpresskraft kann beispielsweise durch eingelagerte Metallbänder oder durch die Härte und/oder Elastizität des Elements **1** und **2** erzeugt und bewirkt werden. Die Endständigen kugelformigen Formkörper **5** verhindern ein herausziehen der flexiblen Elemente **1** und **2** und/oder verbessern die Handhabung dieser.

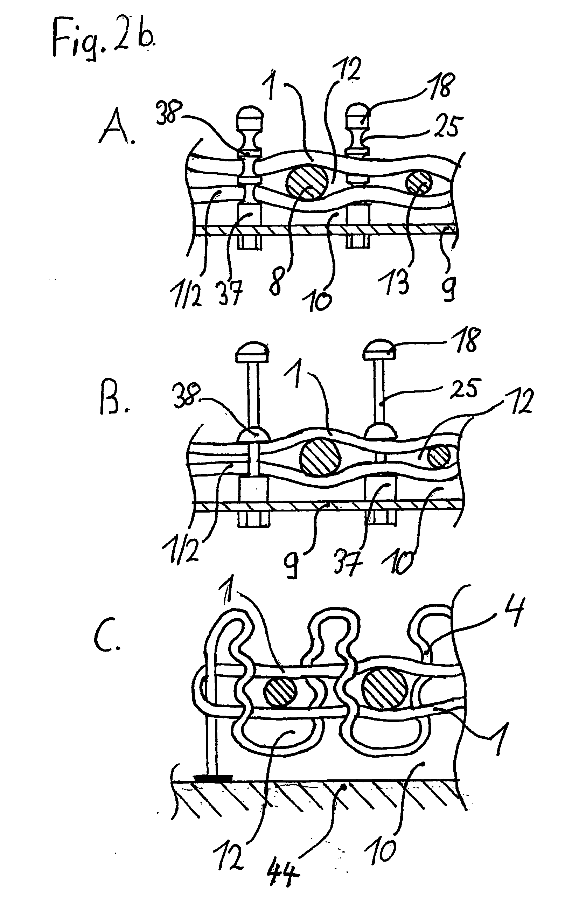

Figur 2b. zeigt detaillierte Ansichten der Erfindung mit zwei beabstandeten eingeflochtenen flexiblen Elementen. Mit A, B und C sind Halterungen gezeigt, die durch die Einflechtung von zwei erfinderischen Elementen hergestellt wurden. Abbildung A. zeigt zwei beabstandete Stifte **25** mit einer abgerundeten Kopfstruktur **18** mit Einkerbungen im Schaftbereich der Stifte **25.** Zwischen den Einkerbungen bildet sich hierbei ein Führungssteil oder -element **38** aus. Ein unteres Band **1** oder **2** bildet hierbei die Produktauflage der Halterung und durch hierzu ein parallel beabstandetes oben eingefädeltes Band **1** bildet die Produktsicherung. Gezeigt ist, wie ein Instrument mit großem Querschnitt **8** mit einem Instrument mit kleinem Querschnitt **13** schwebend gehalten wird. Das untere Band besitzt hierbei einen änderbaren Abstand **10** zur Struktur **9.** Die beiden Bänder oder ein vor- und zurückgeführtes Band **1,** können sich auf Grund der Beabstandung zu einer Struktur **9** die eine Platte oder einer Siebstruktur sein kann, frei nach unten und nach oben bewegen bzw. durch ihre elastischen Eigenschaften ausdehnen. Hierzu befindet sich ein Abstandsteil **37** oder ein Ring im unteren Bereich des Stiftes **25.** Die freien Durchtrittsräume oder -öffnungen **10** und **12** für Medien ändern sich entsprechend den eingelagerten Instrumentenquerschnitten oder über die Höhe und Art der Bandführung **1** und/oder **2** durch die vorgegebnen Stufen bzw. Einkerbungen der Stifte **25.** Abbildung B zeigt wie in der Abbildung A bereits dargestellt, wiederum Stifte **25,** jedoch ohne Einkerbungen oder Stufen im Schaft. Diese Stifte **25** besitzen abgerundete Führungselemente **38** für ein Band **1** oder zwei Bänder **1** und **2.** Auch hier bildet ein unteres Band **1** oder **2** eine Instrumentenauflage und ein oben geführtes Band **1** die Instrumentensicherung. Zwischen beiden Bändern können Instrumente eingeschoben werden. Abbildung C zeigt eine gebogene Gerüststruktur **4,** welche aus einem Draht hergestellt wird. Der Draht **4** wird so gebogen, dass sich hier ein elastisches Element oder Band **1** sicher eingefädelt oder eingebracht werden kann. Das Drahtgerüst **4** ist hier mit einer stabilen Wand **44** verbunden. Vorzugsweise wird das Band **1** vor und zurückgeführt und bildet so eine untere Produktauflage **1.** Ein hier eingeführtes Instrument oder Produkt wird durch das zurückgeführte Band **1** von oben eingeklemmt. Diese erfinderische Ausführung ist in sich elastisch durch das Drahtgerüst **4.** Anstelle des Drahtgerüstes, wäre auch eine Stiftstruktur einsetzbar. Je nach Querschnitt des hier eingelagerten Instrumentes ändert sich die Durchtrittsöffnungen **12** für Medien. Der Abstand **10** zwischen fixiertem Produkt zur Wandoberfläche der Wand **44** ist hierbei nur geringfügig änderbar.

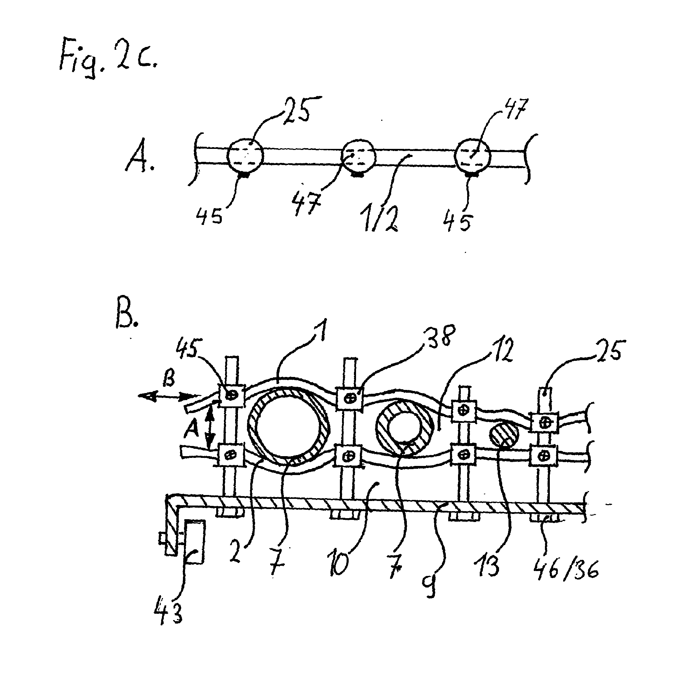

Figur 2c. zeigt Detailansichten von mindestens einem eingebrachten Element in Verbindung mit einer Positionsstruktur. Figur A zeigt eine Draufsicht einer Anordnung von Stiften **25** mit Löchern **47.** Hierbei sind mindestens ein oder zwei flexible Elemente in Form von Bändern **1** oder **2** oder beide in die Löcher **47** der Stifte **25** einer Positionsstruktur eingebracht. Durch Feststellschrauben **45** in den Stiften kann das flexible Element fixiert werden. Diese Art der Stifte eignet sich gut für die Fixierung an eine Wand in senkrechter Position. Figur B zeigt eine praktische Ausgestaltung der Erfindung mit dünnen Stiften **25** mit oder ohne Gewinde, die mit einer Struktur **9** z. B. einem Einschubgestell verbunden ist. Hierbei können die Stifte unten eine Schraubverbindung **36** oder eine Fixierhülse oder nur einen Kopf **46** besitzen. Die Stifte besitzen Führungsteile oder -elemente **38** für die flexiblen Elemente mit einer Feststellschraube **45.** Diese Führungsteile können in der Höhe verstellt und fixiert werden. Die Führungsteile **38** können Löcher für die Aufnahme oder Formstrukturen für die Einflechtung von flexiblen Elementen besitzen. Je nach Größe und Form der hier zu fixierenden oder zu lagernden Produkte **7** oder **13,** kann das flexible Element genau auf die Produktgröße und Form eingestellt werden. Dies geschieht durch die Fixierung über Schrauben **45.** Hierbei wird das erfinderische Element **1** und **2** durch Zug oder durch lockern optimal auf die Produktquerschnitte abgestimmt. Ein einfaches nachjustieren ist nachträglich möglich. Entsprechend ändern sich die Öffnungen **12** in der Halterung oder der Abstand **10** wird zur Oberfläche der Struktur **9** eingestellt. Der **Pfeile A** zeigt hierbei die Höheneinstellung der Führungsteile **38** in Verbindung mit dem Stift **25.** Der **Pfeil B** zeigt, dass die Länge der benötigten flexiblen Elemente **1** und/oder **2** in der Halterung, sich entsprechend der zu fixierenden Produkte oder einzulagernden Produkte, ändern kann. Die Anordnung der Stiftstruktur **25** kann hierbei auch geändert werden, ohne das flexible Band **1** und **2** gewechselt werden muss. In dieser Ausführung können auch ringartige elastische Bandstrukturen verwendet werden. Dadurch, das Rollen **43** an der Struktur **9** angebracht sind, kann diese Vorrichtung als Spülwagen in eine Spülmaschine eingeschoben werden. Die Einführung oder die Entnahme der Produkte geschieht hierbei vorzugsweise durch Einschieben oder Herausschieben zwischen die parallel verlaufenden Bänder **1** und **2.** Alternativ können die Führungsteile **38** oben und unten unterschiedlich ausgeführt und verstellbar sein und damit auch das obere Band entfernbar werden, beispielsweise durch Entflechtung.

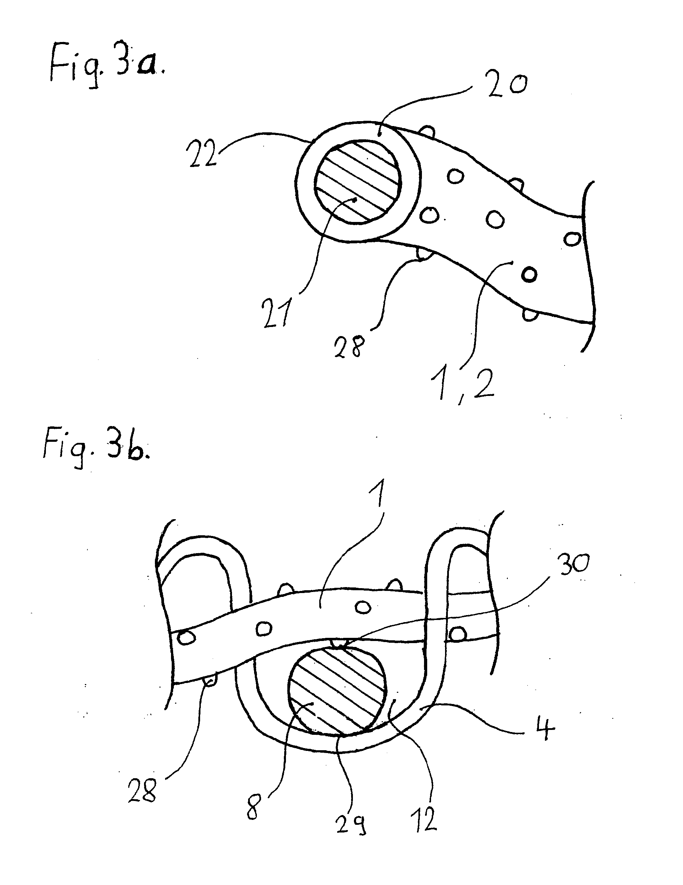

Figur 3a. zeigt eine Schnittdarstellung eines flexiblen Elementes mit einem Kern. In dieser Ausgestaltung des Elementes **1** und/oder **2** besitzt dieser einen flexiblen Kern **21.** Dieser Kern **21** kann beispielsweise eine Kunststoffummantelung **20** besitzen. Als sinnvolle Ummantelung **20** eignet sich z. B. Silikon. Das Kernmaterial **21** kann z. B. aus Metall oder Kunststoff bestehen. Alternativ ein Metalldraht, eine Hohlwelle, eine Glasfaser oder einen Memory-Werkstoff bzw. Material enthalten. Wichtig dabei ist, dass das verwendete Material biegsam ist, damit der Anwender dieses Element manuell in die Gerüststruktur einer Halterung einflechten kann. Ein flexibles Element, dass aus einem Memoy-Werkstoff besteht oder diesem im Kern **21** oder in der Ummantellung **20** besitzt, hat den Vorteil, dass über Zustandsänderungen die Eigenschaften des flexiblen Elementes veränderbar sind. Das flexible Element kann auf seiner Oberfläche **22** eine Oberflächenstruktur oder eine Beschichtung besitzen, welche z. B. die Hafteigenschaften verbessert. Die Oberflächenstrukturierung auf der Oberfläche **22** kann, wie hier gezeigt, als Noppenstruktur **28** ausgebildet sein, die sich gleichmäßig über die Oberfläche **22** erhebt. Alternativ kann die Oberfläche z. B. kleinste Häckchen, ähnlich wie bei einem Klettverschluss oder eine faserartige Aufrauung besitzen, um die physikalische Verbindung mit einer Gerüststruktur zu verstärken. Alternativ können Nanostrukturierungen oder Nanobeschichtungen technisch aufgebracht sein.

Figur 3b. zeigt eine detaillierte Schnittdarstellung einer Drahthalterung als Gerüststruktur mit einem Produkt in einer 1-Punkt-1-Flächen-Lagerungsposition. In der Drahthalterung **4** befindet sich z. B. ein medizinisches Instrument **8.** Das flexible Element **1** besitzt Noppenstrukturen **28** oder ähnliche Oberflächenerhebungen und ist in die Drahthalterung **4** so eingeflochten, dass es das Instrument **8** einklemmt. Im oberen Bereich liegt das Instrument **8** punktuell **30** an dem flexiblen Element an und im unteren flächig **29** an der Drahthalterung **4.** Es ist eine 1-Punkt-1-Flächen-Lagerung entstanden. Im Halterungsbereich ist der Öffnungsbereich **12** gut zu sehen, durch das Lösung hindurchströmen kann. Dieser Öffnungsbereich **12** vergrößert sich, wenn der Abstand des Elements **1** zur Produktoberfläche vergrößert wird. Aus der 1-Punkt-1-Flächen-Lagerung entsteht dann eine 1-Fiächen-Lagerung.

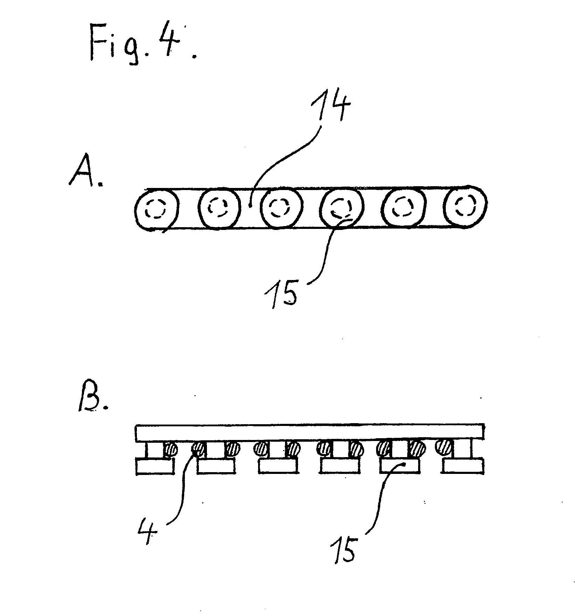

Figur 4. zeigt ein schematisches flexibles Element mit zusätzlichen funktionalen Formelementen. Abbildung A zeigt das Element als ein flaches Band **14,** welches an seiner Stirnfläche knopfartige runde Formelemente **15** besitzt. Das Formelement **15** ist mit einem Steg mit **14** verbunden. Diese Ausbildung des flexiblen Elementes hat den Vorteil, dass sich die Formelemente in ein Drahtgerüst verankern lassen und so ein Herausrutschen verhindert wird. Abbildung B zeigt eine Draufsicht in Schnittdarstellung, bei dem das Formelement **15** des elastischen Bandes **14** eine physikalische Verbindung z. B. eine Pressverbindung mit einem Drahtgerüst einer Halterung **4,** eingeht. Hierbei wird die Vorrichtung z. B. von oben in die Gerüststruktur der Halterung eingepresst. Die Formelemente **15** drücken gegen das Drahtgerüst **4** und nach hinten erfolgt ein flächiger Kontakt mit der Stirnseite des elastischen Elementes **14.**

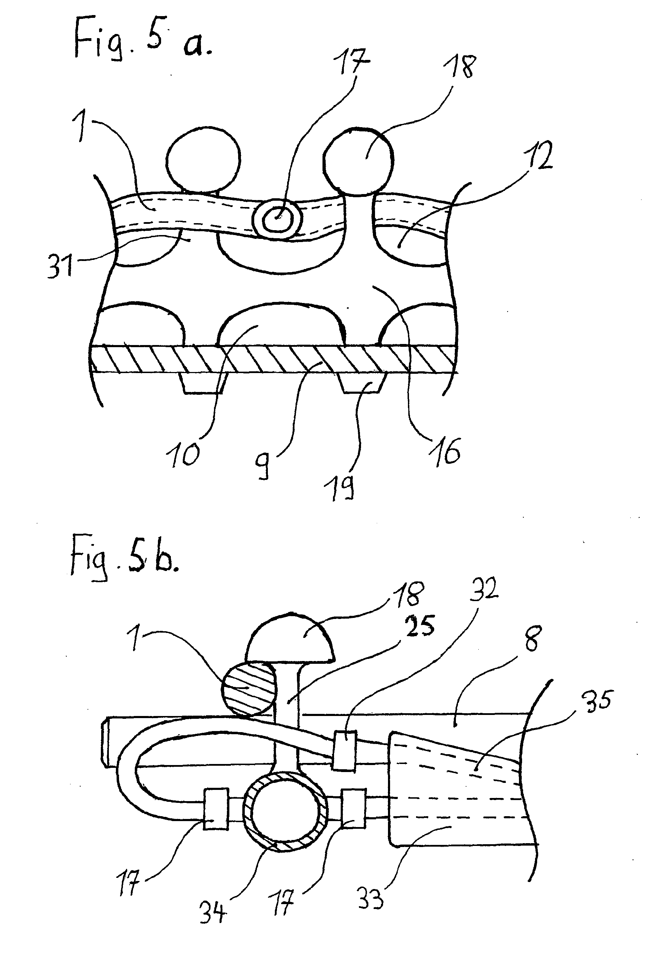

Figur 5a. zeigt eine detaillierte Schnittdarstellung einer Gerüststruktur eines Halters mit einem flexiblen Element zum Durchspülen von Instrumenten. Die hier gezeigte Gerüststruktur **16** der Halterung besitzt eine Kopfstruktur **18** z. B. als Kugelkopf in Verbindung mit einen Steg **31.** In die obigen Aussparungen kann z. B. ein elastisches Element **1** individuell eingeflochten werden. Alternativ kann dieses Element eine bereits teilweise vorgeformte Form besitzen. Die Kopfstruktur **18** verhindert, dass das elastische Element **1** zu leicht aus der Verankerung herausrutscht. Das flexible Element **1** ist hier als ein Schlauch oder Röhre dargestellt und besitzt Anschlüsse **17.** An diesen Anschlüssen **17,** die alternativ z. B. auch ein Adapter, ein Konnektor oder ein Ventil sein können, kann z. B. ein medizinisches Instrument angeschlossen sein und direkt mit Medien durchspült werden. Der Vorteil dieser Anordnung ist, das zerlegbare medizinische Instrumententeile in einem Halterungssystem fixierbar sind und Instrumentenhohlräume oder Kanäle über Anschlüsse **17** am flexiblen Element **1** mit Medien durchströmt werden können. Die Gerüststruktur **16** der Halterung kann beispielsweise aus Edelstahl oder aus einem temperaturstabilen Kunststoff hergestellt sein. Alternativ kann die Gerüststruktur punktuelle Erhöhungen im Halterungsbereich besitzen, damit eine Punkt-Lagerung mit einem entsprechend ausgeformten flexiblem Element möglich ist. Mit einer Steckverbindung **19** kann diese an eine Platte **9,** z. B. in Form einer Lochplatte, befestigt werden. Die Gerüststruktur **16** besitzt im unteren Teil Ausformungen oder Öffnungen **10.** Im oberen Teil sind die Öffnungen **12** ebenfalls groß genug, damit Medien und Wellen die Vorrichtung umströmen oder stirnseitig durchströmen können.

Figur 5b. zeigt eine detaillierte Schnittdarstellung einer Spülleiste zum Durchspülen von Instrumenten mit einem flexiblen Element als Instrumentensicherung. Eine Spülleiste **34** bestehend z. B. aus einer Edelstahlröhre besitzt links und rechts Anschlüsse oder Adapter **17** für Instrumente **33.** Alternativ kann sich ein Adapter **17** im Kopfbereich **18** oder am Positionsstift **25** befinden. Das hier gezeigte Instrument **33** besitzt zwei Innenkanäle **35.** Diese müssen beim Reinigen durchspült werden. Das Instrument **33** ist einerseits über einen Adapter **17** direkt mit der Spülleiste **34** verbunden und anderseits über einen Schlauch mit einer Speckverbindung **32.** Ein anderes Instrument **8** ist über ein flexibles Element **1** vor dem Herausfallen gesichert. Als Herausrutschschutz befindet sich im Kopfbereich, mit einem Stift **25** verbunden, eine pilzähnliche Kopfstruktur **18.** Das flexible Element **1** ist entfernbar. Alternativ könnte auch wie in Figur 5a gezeigt, ein Instrumentenkanal über einen Adapter am flexiblem Schlauchelement **1** das Instrument **33** mit Medium versorgen.

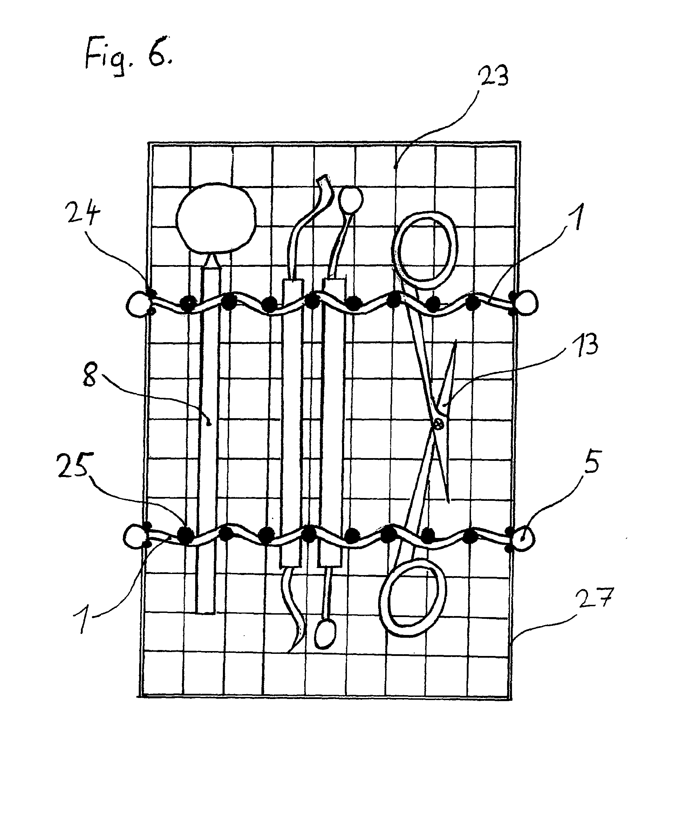

Figur 6. zeigt die Draufsicht einer Siebplatte mit fixierten geometrisch angeordneten Positionsstiften bestückt mit chirurgischen Instrumenten. Das flexible Element **1** in Ausformung eines Bandes oder Schnur fixiert in dieser Ausführung medizinische Instrumente aus dem Dentalbereich. Die dargestellten Instrumente **8** mit rundem Querschnitt und eine Schere **13** liegen direkt auf ein Metallgitter **23** auf. Alternativ kann anstelle eines Metallgitters **23** auch eine Platte z. B. aus Kunststoff oder ein Instrumentensieb die Unterlage bilden. Das Metallgitter besitzt zur Stabilisierung eine Metallumrandung **27.** Mit der grobmaschigen Gitterstruktur **23** verbunden befinden sich Positionsstifte **25** mit einem Kugelkopf. Diese stellen die Gerüststruktur der Instrumentenhalter dar. Anstelle des Kugelkopfes als Formelement können alternativ andere Strukturen wie z. B. Ankerstrukturen als Positionsstifte **25,** verwendet werden. Um die Instrumente auf der Unterlage zu fixieren, wird das flexible Band **1** in wellen um die Positionsstifte **25** geführt. Es entsteht eine 2-Punkt-Lagerung mit einem Auflagepunkt zum Gitter hin und ein Auflagepunkt zum flexiblen Band. Endständig besitzen die biegsamen und elastischen Elemente ein kugeliges Formteil **5** zur Einklemmung in eine Klemmhalterung **24.** Durch herauslösen der endständigen Formelemente **5** des flexiblen Bandes **1** aus der Klemmhalterung **24** können die Instrumente frei entnommen werden. Erfindungsgemäß kann ein zusätzliches flexibles Element **1** als Auflage für die Instrumente eingesetzt werden, falls der direkte Kontakt der Instrumente mit dem Metallgitter **23** diese schädigt. Die Positionsstifte **25** müssen in diesem Fall nur entsprechend lang ausgelegt sein. Diese Alternative ermöglicht eine höhenverstellbare Halterungs- oder Lagerungsflächen.

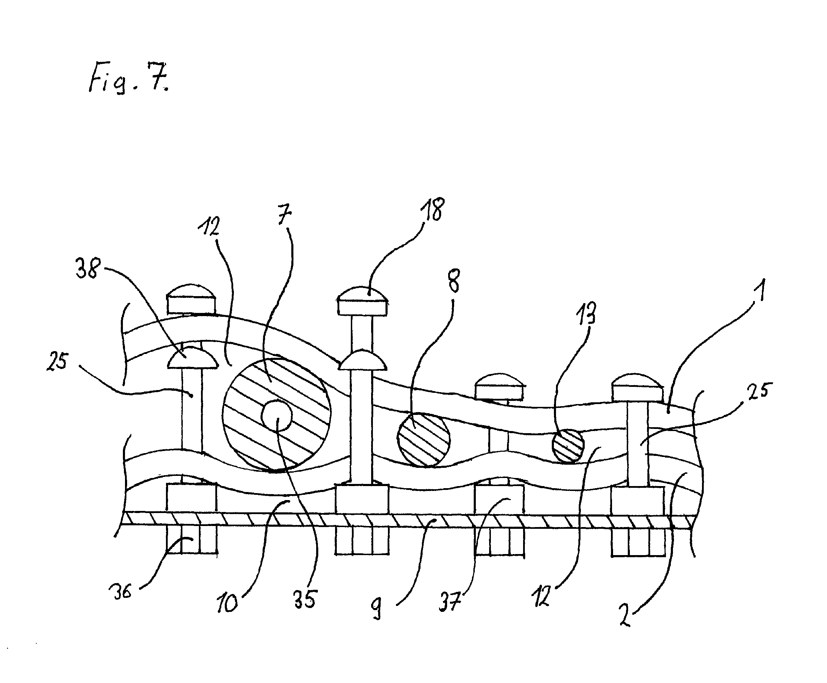

Figur. 7. zeigt eine Schnittdarstellung einer Halterung bei der das Element eine flexible Produktlagerung und -sicherung in Kombination mit Positionsstiften ermöglicht. Das flexible Element **2** bildet in Ausformung eines Silikonbandes die Produktauflage. Ein zweites beabstandetes oberes flexibles Element **1** in Ausformung eines Silikonbandes bildet die Produktsicherung. Beide Bänder **1** und **2** deren Durchmesser verschieden sein kann, bilden zusammen mit den unterschiedlich hohen und ausgebildeten Positionsstrukturen in Form von Positionsstiften **25** die Halterung. Diese kann insbesondere von Medien gut um- und durchspült werden. Die Positionsstifte **25** besitzen als Abschluss einen Rundkopf **18** und können z. B. ein abgerundetes Führungsteil **38** im Schaftbereich aufweisen. Das Führungsteil **38** sorgt für eine sichere Führung oder die Führungsrichtung des oberen Elements **1** innerhalb der Positionsstifte **25** und unterstützt so die Fixierung von unterschiedlich großen Produktquerschnitten. Hier können je nach Länge des Schaftes mehrere derartige Führungsteile **38,** auch in anderer Ausformung, vorhanden sein. Damit das untere flexible Element nicht auf die Oberfläche der Struktur **9** aufliegt oder aufliegen kann, besitzt der Halterungsstift einen unteren Abstandsring **37.** Durch Verschiebung nach oben oder durch die Ausführungshöhe des Abstandsringes **37,** erlaubt dieser eine Abstandserhöhung bzw. Durchgang für Medien **10** des flexiblen Elements **2** zur Oberfläche der Struktur **9.** Damit wird ein größerer untere Durchgang **10** z. B. für Medien geschaffen und der Öffnungsbereich **12** in der Halterung verändert. Mit dieser Anordnung zweier Elemente **1, 2** oder auch eines vor und zurücklaufenden flexiblen Elementes **2** wird eine Einklemmung von unterschiedlich Instrumenten **7, 8 13** mit großen Querschnittsdurchmessern ermöglicht. Hierbei können auch große Instrumente **7** mit innenliegenden Kanälen **35** wie z. B. Handstücke aus der Augenchirurgie positioniert werden. Die Positionsstifte **25** sind hier z. B. mit Schrauben **36** an eine Struktur **9** fixiert und können im Falle einer Lochplatte individuell oder Produktbezogen versetzt werden. Damit ändert sich die Positionsstruktur und damit die Halterungsstruktur wie z. B. die Produktauflagefläche und Spannweite der Fixierung wobei das Element z. B. individuell zwischen den Positionsstiften geführt wird.

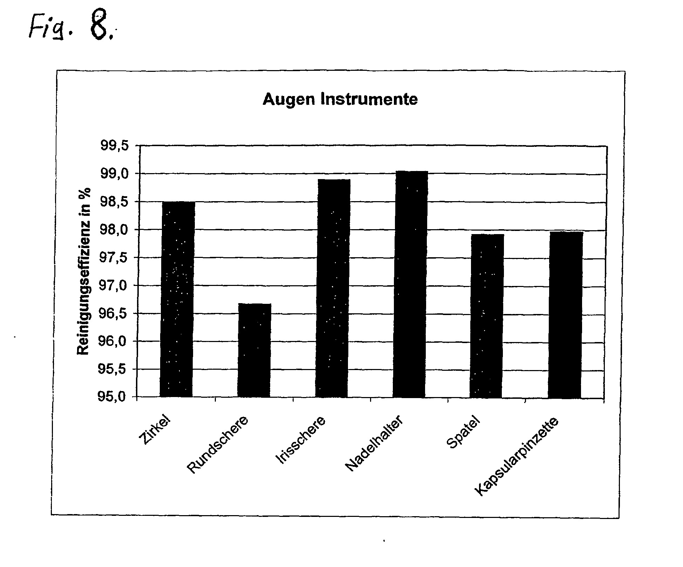

Figur 8. zeigt die Reinigungseffizienz von Augeninstrumenten die mit der erfinderischen Bandhalterung gelagert wurden. Das gemittelte Ergebnis aus mehren Experimenten zeigt, dass schwebend gelagerte Instrumente die zwischen einem oberen und einem unteren Silikonband eingeklemmt wurden eine Reinigungseffizienz von über 95 % besitzen. Das Reinigungsergebnis basiert auf die hoch empfindliche und validierte Radionuklidmethode in Verbindung mit einer radioaktiv markierten Testanschmutzung. Silikonhalterungen der bekannten Art zeigen ein nicht so gutes Reinigungsverhalten, da hier größere Spülschatten durch die flächig aufliegenden Silikonstreifen entstehen. Die Erfindung unterstützt damit Reinigungsverfahren von Instrumenten durch geeignete Lagerung. Die Produktaufbereitung bzw. das Reinigungsverfahren wird verbessert, da die Reinigungsmedien aber auch die Sterilitätsmedien die schwebend gelagerten Instrumente fast vollständig umströmen können.

## Patentansprüche

1. Verwendung mindestens eines flexiblen Elements zur Fixierung eines Produktes insbesondere für die Aufbereitung, Behandlung, Bereitstellung und Transport von Medizinprodukten, **dadurch gekennzeichnet, dass** über das mindestens eine flexible Element (1, 2, 14) in Verbindung mit einer Gerüst-, Positions- o- der Halterungsstruktur (4, 16, 25) die Produktfixierung und/oder Lagerung änderbar ist und damit die Produktbehandlung und -handhabung und/oder Produktbereitstellung unterstützt oder beeinflussbar wird, und
dass mit einem unteren flexiblen Element oder einem unteren Teil eines einzigen flexibten Elements (1, 2, 14) als Auflage und mit einem beabstandeten oberen flexiblen Element oder einem zurückgeführten oberen Teil des einzigen flexibten Elements (1, 2, 14) als Sicherung die Produktlagerung höhenverstellbar, beweglich, nachjustierbar und/oder einstellbar ist.

2. Verwendung nach Anspruch 1, **dadurch gekennzeichnet, dass** durch mindestens ein flexibles Element (1, 2, 14) zusammen mit der Gerüst, Positions- oder Halterungsstruktur (4, 16, 25) mindestens eine flächige (29) und/oder mindestens eine punktuelle (30) Produktlagerung und damit eine Reduzierung des Reinigungsschattens bei der Reinigung von Produkten bewirkt und/oder die Desinfizierung, Sterilisierung und/oder die Trocknung und/oder die Fixierung von Produkten verbessert wird.

3. Verwendung nach den Ansprüchen 1 bis 2, **dadurch gekennzeichnet, dass** unterschiedlich große Produktdurchmesser und -querschnittsformen mit oder ohne Führungsteile (38) feststellbar gelagert, fixiert oder gesichert werden.

4. Verwendung nach den Ansprüchen 1 bis 3, **dadurch gekennzeichnet, dass** sich zwischen dem mindestens einem flexiblen Element (1, 2, 14) und der Gerüst- Positions- oder Halterungsstruktur (4, 16, 25) änderbare durchgängige Öffnungen (12) für Medien oder physikalische Wellen befinden.

5. Verwendung nach den Ansprüchen 1 bis 4, **dadurch gekennzeichnet, dass** durch mindestens ein unteres flexible Element (1, 2, 14) in Verbindung und der Gerüst- Positions- oder Halterungsstruktur (4, 16, 25) die Produkte mit Abstand oder über Öffnungen (10) über eine Struktur (9, 23, 34, 42) fixierbar sind.

6. Verwendung nach den Ansprüchen 1 bis 5, **dadurch gekennzeichnet, dass** durch Zug oder manuelles Justieren des flexiblen Elements (1, 2, 14) die Stabilität der Produktlagerung oder -beweglichkeit änderbar ist

7. Verwendung nach den-Ansprüchen 1 bis 6, **dadurch gekennzeichnet, dass** über mindestens ein flexibles Elements (1, 2, 14) Medien oder physikalische Wellen zur Produktbehandlung eingebracht werden.

8. Verwendung nach den Ansprüchen 1 bis 7, **dadurch gekennzeichnet, dass** über mindestens ein flexibles Element (1, 2, 14) oder über ein flexibles Element (1, 2, 14) in Verbindung mit einer Spülleiste Produkte mit Kanälen oder Röhren durchspült werden.

9. Verwendung nach den Ansprüchen 1 bis 8, **dadurch gekennzeichnet, dass** Produkte durch Entfernung des oberen flexiblen Elements (1, 2, 14) oder durch herausschieben entnommen werden und dabei sterile Produkte steril bleiben.

10. Verwendung nach den Ansprüche 1 bis 9, **dadurch gekennzeichnet, dass** durch elektronische Komponenten in oder an einem flexiblen Element (1, 2, 14) die Produktbehandlung in Maschinen gesteuert und/oder damit Behandlungsereignisse und -abläufe dokumentiert werden.

11. Verwendung nach einem der Anspruche 1-10, **dadurch gekennzeichnet, dass** das flexibte Elements die Form eines Bandes, eines Drahtes oder eines Kabels besitzt

12. Verwendung nach einem der Anspruche 1-10, **dadurch gekennzeichnet, dass** das flexibte Elements die Form einer Hohlwelle, einer Röhre oder eines Schlauches besitzt.

13. Verwendung nach einem der Anspruche 1-10, **dadurch gekennzeichnet, dass** das flexibte Elements mindestens ein endständiges Formteil (5) in Form einer Hülse, Kugel oder eines Labels aufweist.

14. Verwendung nach einem der Anspruche 1-13, **dadurch gekennzeichnet, dass** die Einflechtung und Einpassung oder Einbringung und/oder die Länge des flexibtene Elements durch Abschneiden änderbar ist und dass flexible Elemente unterschiedlichen Durchmessers und Querschnittsformen miteinander kombinierbar sind.

15. Verwendung nach einem der Anspruche 1-14, **dadurch gekennzeichnet, dass** das flexibte Elements an der Oberfläche Noppenstrukturen (28), Ausformungen (15), faserartige Aufrauungen, punktuelle Oberflächenerhe6ungen oder Beschichtungen aufweist.

16. Verwendung nach einem der Anspruche 1-15, **dadurch gekennzeichnet, dass** das flexibte Elements fest verbundene oder austauschbare und/oder verschiebbare Formteile (5) besitzt und in Verbindung mit einem Halter (24, 39) oder einer Schraube fixiert oder eingeklemmt wird und/oder das endständig Label in Form von Transponder angebracht sind.

17. Verwendung nach einem der Anspruche 1-16, **dadurch gekennzeichnet, dass** das flexibte Elements aus einem oder mehreren Materialien mit oder ohne Kern (21) besteht.

18. Verwendung nach Anspruch 17 **dadurch gekennzeichnet, dass** der Kern (21) Hohlwellen, Metalldrähte, Glasfasern Stromleiter oder andere biegsame Teile und/oder elektronische Komponenten enthält.

19. Verwendung nach Anspruch 17 **dadurch gekennzeichnet, dass** das flexibte Element aus blegsamen und/oder elastischen und/oder temperaturstabilen Materialien aus den Bereichen der Metalle, der Kunststoffe, der Glasstoffe oder der Memory-Materiallen besteht

20. Verwendung nach Anspruch 17 **dadurch gekennzeichnet dass** das flexibte Element aus farbigen oder transparenten Material mit oder ohne flureszierenden Farben und/oder aus einer oder unterschiedlichen Materialhärten besteht

## Claims

1. Use of at least one flexible element for fixing a product, in particular for reprocessing, handling, supplying and transporting medical products,
**characterized in that** by means of the at least one flexible element (1, 2, 14) in combination with a rack, positioning or supporting structure (4, 16, 25) the product fixing and/or support is modifiable and therefore the product treatment and handling and/or supplying the product is supported or becomes controllable, and **in that** the product support is adjustable in height, moveable, readjustable and/or adjustable with the aid of a lower flexible element or a lower portion of a single flexible element (1, 2, 14) as a support and with a spaced upper flexible element or a returned upper portion of the single flexible element (1, 2, 14) as a securing means.

2. The use according to claim 1, **characterized in that** by using at least one flexible element (1, 2, 14) together with the rack, positioning or supporting structure (4, 16, 25) at least one flat (29) and/or at least one point-shaped (30) product support and therefore a reduction of the cleaning shadow during cleaning of products is caused and/or the disinfection, sterilisation and/or the drying and/or the fixing of products is improved.

3. The use according to claims 1 to 2, **characterized in that** different product diameters and cross-sectional shapes with or without guiding members (38) are supported in a fixable manner, or fixed or secured.

4. The use according to claims 1 to 3, **characterized in that**, between the at least one flexible element (1, 2, 14) and the rack, positioning or supporting structure (4, 16, 25) modifiable continuous openings (12) are present for media or physical waves.

5. The use according to claims 1 to 4, **characterized in that** the products are fixable in a spaced relationship or above openings (10) above a structure (9, 23, 43, 42) by means of at least one lower flexible element (1, 2, 14) in combination with the rack, positioning or supporting structure (4, 16, 25).

6. The use according to claims 1 to 5, **characterized in that** the stability of the product support or mobility is modifiable by tensile loading or manual adjustment of the flexible element (1, 2, 14).

7. The use according to claims 1 to 6, **characterized in that** media or physical waves are introduced for product treatment via at least one flexible element (1, 2, 14).

8. The use according to claims 1 to 7, **characterized in that** products with channels or tubes are flushed via at least one flexible element (1, 2, 14) or via a flexible element (1, 2, 14) in combination with a flushing manifold.

9. The use according to claims 1 to 8, **characterized in that** products are removed by removing the upper flexible element (1, 2, 14) or by sliding out whereby sterile products remain sterile.

10. The use according to claims 1 to 9, **characterized in that** product treatment in machines is controlled and/or treatment events and processes are documented by means of electronic components in or on a flexible element (1, 2, 14).

11. The use according to claims 1 to 10, **characterized in that** the flexible element has the form of a band, a wire or a cable.

12. The use according to claims 1 to 10, **characterized in that** the flexible element has the form of a hollow shaft, a tube or a hose.

13. The use according to claims 1 to 10, **characterized in that** the flexible element includes at least one moulded part (5) on the end in the form of a sleeve, a sphere or a label.

14. The use according to claims 1 to 13, **characterized in that** weaving-in and fitting or introduction and/or the length of the flexible element is modifiable by means of cutting and **in that** flexible elements of different diameters and cross-sectional shapes can be combined with one another.

15. The use according to claims 1 to 14, **characterized in that** the flexible element has nub structures (28), shaped structures (14), fibrous roughnesses, raised surface points or coatings on its surface.

16. The use according to claims 1 to 15, **characterized in that** the flexible element comprises fixedly joined or exchangeable und/or slidable moulded parts (5) and is fixed or clamped in combination with a holder (24, 39) or a screw and/or the label on the end is attached in the form of a transponder.

17. The use according to claims 1 to 16, **characterized in that** the flexible element consists of one or more materials with or without a core (21).

18. The use according to claim 17, **characterized in that** the core (21) contains hollow shafts, metal wires, glass fibres, electric conductors or other flexible parts and/or electronic components.

19. The use according to claim 17, **characterized in that** the flexible element consists of flexible and/or resilient and/or temperature-resistant materials from the fields of metals, plastic materials, glass materials or memory materials.

20. The use according to claim 17, **characterized in that** the flexible element consists of a pigmented or transparent material with or without fluorescent pigments and/or of the same or different material hardnesses.

## Revendications

1. Utilisation d'au moins un élément flexible pour la fixation d'un produit, en particulier pour la préparation, le traitement, la mise à disposition et le transport de produits médicaux, **caractérisée en ce que,** par l'intermédiaire d'au moins un élément flexible (1, 2, 14) en relation avec une structure d'ossature, de positionnement ou de support (4, 16, 25), la fixation du produit et/ou le stockage peut (peuvent) être modifié(s), la manutention et le maniement du produit et/ou sa mise à disposition sont soutenus ou peuvent être influencés, et **en ce que**, avec un élément flexible inférieur ou une partie inférieure d'un élément flexible unique (1, 2, 14) comme appui et avec un élément flexible supérieur distant ou une partie supérieure ramenée de l'élément flexible unique (1, 2, 14) comme sûreté, le stockage du produit est réglable en hauteur, déplaçable, réajustable et/ou réglable.

2. Utilisation selon la revendication 1, **caractérisée en ce que** par au moins un élément flexible (1, 2, 14) associé avec la structure d'ossature, de positionnement ou de support (4, 16, 25), au moins un stockage du produit en nappe (29) et/ou au moins un stockage du produit ponctuel (30) et donc, une diminution de la surface de nettoyage est obtenue lors du nettoyage des produits et/ou la désinfection, la stérilisation et/ou le séchage et/ou la fixation du produit est(sont) amélioré(s).

3. Utilisation selon les revendications 1 à 2, **caractérisée en ce que** des diamètres et des formes de produit de grandeurs différentes sont stockés, fixés ou assurés de manière constatable avec ou sans éléments de guidage (38).

4. Utilisation selon les revendications 1 à 3, **caractérisée en ce que,** entre au moins un élément flexible (1, 2, 14) et la structure d'ossature, de positionnement ou de support (4, 16, 25), des ouvertures interconnectées modifiables (12) pour des agents où des arbres physiques sont présents.

5. Utilisation selon les revendications 1 à 4, **caractérisée en ce que** par au moins un élément flexible inférieur (1, 2, 14) en liaison et la structure d'ossature, de positionnement ou de support (4, 16, 25), les produits peuvent être fixés à espacement ou par des ouvertures (10) au-dessus d'une structure (9, 23, 34, 42).

6. Utilisation selon les revendications 1 à 5, **caractérisée en ce que** par traction ou par ajustement manuel de l'élément flexible (1, 2, 14), la stabilité du stockage ou de la mobilité des produits est modifiable.

7. Utilisation selon les revendications 1 à 6, **caractérisée en ce que** via au moins un élément flexible (1, 2, 14), des agents ou des arbres physiques peuvent être introduits pour le traitement du produit.

8. Utilisation selon les revendications 1 à 7, **caractérisée en ce que** via au moins un élément flexible (1, 2, 14) ou via un élément flexible (1, 2, 14) en relation avec un listel de rinçage, des produits sont rincés au moyen de canaux ou de tuyaux.

9. Utilisation selon les revendications 1 à 8, **caractérisée en ce que** des produits sont prélevés par enlèvement de l'élément flexible supérieur (1, 2, 14) ou par extraction, des produits stériles restant ainsi stériles.

10. Utilisation selon les revendications 1 à 9, **caractérisée en ce que** par des composants électroniques dans un élément flexible (1, 2, 14) ou sur celui-ci, le traitement des produits en machine est commandé et/ou des événements et des déroulements de traitement sont ainsi documentés.

11. Utilisation selon l'une des revendications 1 à 10, **caractérisée en ce que** l'élément flexible a la forme d'une bande, d'un fil ou d'un câble.

12. Utilisation selon l'une des revendications 1 à 10, **caractérisée en ce que** l'élément flexible a la forme d'un arbre creux, d'un tuyau (rigide) ou d'un tuyau flexible.

13. Utilisation selon l'une des revendications 1 bis 10, **caractérisée en ce que** l'élément flexible présente au moins un élément de forme terminal (5) sous la forme d'un manchon, d'une bille ou d'un label.

14. Utilisation selon l'une des revendications 1 à 13, **caractérisée en ce que** l'entrelacement, et l'intégration ou l'introduction et/ou la longueur de l'élément flexible est modifiable par découpage et que des éléments flexibles aux diamètres et aux formes différents sont combinables mutuellement.

15. Utilisation selon l'une des revendications 1 à 14, **caractérisée en ce que** l'élément flexible présente à la surface des structures à nopes (28), des formages (15), des rugosités fibreuses, des élévations de surface ponctuelles ou des enductions.

16. Utilisation selon l'une des revendications 1 à 15, **caractérisée en ce que** l'élément flexible possède des éléments de forme reliés fixement ou interchangeables et/ou déplaçables (5) et en relation avec un support (24, 39) ou une vis est fixé ou enserré et/ou le label terminal est appliqué sous la forme d'un transpondeur.

17. Utilisation selon l'une des revendications 1 à 16, **caractérisée en ce que** l'élément flexible est constitué d'un ou de plusieurs matériaux avec ou sans noyau (21).

18. Utilisation selon la revendication 17, **caractérisée en ce que** le noyau (21) contient des arbres creux, des fils métalliques, des fibres de verre, des conducteurs ou d'autres parties souples et/ou composants électroniques.

19. Utilisation selon la revendication 17, **caractérisée en ce que** l'élément flexible est constitué de matériaux souples et/ou élastiques et/ou thermorésistants provenant des domaines des métaux, des plastiques, des matières vitreuses ou des matériaux à mémoire.

20. Utilisation selon la revendication 17, **caractérisée en ce que** l'élément flexible est constitué d'un matériau coloré ou transparent avec ou sans couleurs fluorescentes et/ou de duretés de matériaux uniques ou différentes.
